(19) Europäisches Patentamt
European Patent Office
Office européen des brevets

(11) EP 2 558 024 B1

(12) **EUROPEAN PATENT SPECIFICATION**

(45) Date of publication and mention
of the grant of the patent:
**08.03.2017 Bulletin 2017/10**

(21) Application number: **11769475.2**

(22) Date of filing: **12.04.2011**

(51) Int Cl.:
***A61F 2/02*** *(2006.01)*

(86) International application number:
**PCT/US2011/032182**

(87) International publication number:
**WO 2011/130322 (20.10.2011 Gazette 2011/42)**

(54) **MACROPOROUS BIOENGINEERED SCAFFOLDS FOR CELL TRANSPLANTATION**

MAKROPORÖSE, BIOTECHNOLOGISCHE GERÜSTE ZUR ZELLTRANSPLANTATION

ÉCHAFAUDAGES BIOTECHNOLOGIQUES MACROPOREUX POUR LA TRANSPLANTATION DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2010 US 342377 P**

(43) Date of publication of application:
**20.02.2013 Bulletin 2013/08**

(73) Proprietors:
• **The University Of Miami**
  **Miami, FL 33136 (US)**
• **Converge Biotech Inc.**
  **Miami FL 33132 (US)**

(72) Inventors:
• **ANDERSON, Cheryl Stabler**
  **Coral Gables**
  **Florida 33146 (US)**
• **PEDRAZA, Eileen**
  **Coral Gables**
  **Florida 33134 (US)**

• **FRAKER, Christopher A.**
  **Hollywood**
  **Florida 33021 (US)**
• **RICORDI, Camillo**
  **Miami**
  **Florida 33133 (US)**
• **HUBBELL, Jeffrey**
  **CH-1028 Preverenges (CH)**
• **WEAVER, Jessica**
  **Miami**
  **Florida 33173 (US)**

(74) Representative: **Vossius & Partner**
**Patentanwälte Rechtsanwälte mbB**
**Siebertstrasse 3**
**81675 München (DE)**

(56) References cited:
**EP-A1- 1 466 633          WO-A1-98/05304
WO-A2-2008/075339     WO-A2-2008/075339
WO-A2-2010/030714     US-A1- 2008 044 900
US-A1- 2008 044 900    US-A1- 2009 149 569
US-A1- 2009 163 612**

**Description**

BACKGROUND

Cell replacement therapy is a promising potential treatment option for a wide variety of diseases. Many clinical conditions and disease states result from the lack of factor(s) produced by living cells or tissues, including, for example, diabetes, in which insulin production is inadequate; Parkinson's disease, in which dopamine production is decreased; and anemia, in which erythropoietin is deficient. Such conditions or diseases may be treated by cell/tissue implants that produce the missing or deficient factor(s).

However, many challenges remain in the field of cell replacement therapy. The viability and functionality of transplanted cells is compromised by, for example, lack of mechanical protection, lack of necessary factors/nutrients (e.g., due to inadequate vascularization or inability of the vascular system to reach parts of the transplant), and inflammatory responses. Thus, there is a need for methods and devices that optimize the viability and functionality of implanted cells.

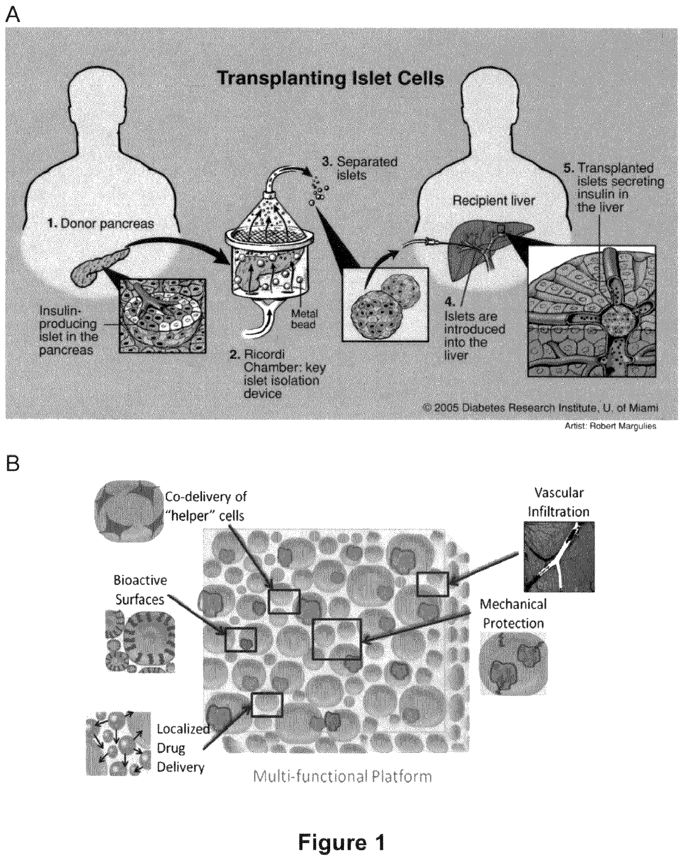

Type 1 diabetes mellitus (T1DM) is an autoimmune disorder characterized by the destruction of insulin producing beta cells found in the islets of Langerhans of the pancreas. Clinical transplantation of insulin-producing cells offers a solution to restoring beta cell function through the intrahepatic transplantation of, e.g., allogeneic pancreatic islets into a diabetic recipient (see Fig. 1A). Such transplantation can lead to improved control in blood glucose levels, higher C-peptide levels, and insulin independence for several years, delaying the onset and reducing the intensity of diabetes-related complications. However, the success of clinical insulin-producing cell transplantation is hindered by the high rate at which transplanted cells are destroyed and/or rendered nonfunctional. This is determined in part by the standard intrahepatic location of the implant site, which is prone to mechanical stresses and inflammatory responses, is exposed to high drug and toxin loads, and renders the transplanted cells irretrievable. Transplantation of insulin-producing cells at alternative sites, such as the subcutaneous space, may alleviate many of these issues. However, relocating insulin-producing cells to alternative sites requires adequate mechanical protection and spatial distribution of the transplanted cells, as well as access to a fully developed vasculature.

SUMMERY OF THE INVENTION

To address these issues, we have designed and developed a highly porous organosilicone (polydimethylsiloxane, PDMS) scaffold capable of providing structural support and adjustable spatial distribution to cells without hindering nutrient delivery. The scaffold provides a superior, more physiological environment for the cells, leading to improved viability and function. Furthermore, the scaffold material itself is modified to provide sustained delivery of biologically active agents that improve the engraftment, survival, function and long-term viability of the cells. Such agents include, but are not limited to, growth factors or growth-stimulating factors and opt. oxygen generating, releasing or transport-enhancing agents, anti-inflammatory compounds and immunosuppressive agents. Table 1 (below) indicates certain advantages of the scaffold and select reasons for their importance.

| Table 1 | |
|---|---|
| **Desired Parameters** | **Reason** |
| Biocompatibility and biostability | Future retrievability |
| High degree tortuosity | High cell retention and size-adjustable homogenous spatial distribution |
| Varied pore size | |
| High degree of porosity | Maximize exchange of nutrient and waste |
| High diffusivity of oxygen | |
| Large pore size | Promote vascular infiltration |

The implanted cells may be, e.g., insulin-producing cells. Structural support to insulin-producing cells in the form of a scaffold is critical to reducing pelleting and agglutination of the cells, which results in decreased availability of nutrients to the cells and thus leads to cell death. The highly porous organosilicone scaffold maximizes nutrient delivery by creating a structure that supports and spatially distributes the insulin-producing cells, and also promotes vascular infiltration.

Highly porous, biocompatible and biostable scaffolds within alternative transplantation sites offer a rational strategy for improving overall cell engraftment (Fig. 1B). These scaffolds provide mechanical protection to the cells, afford retrievability, and for cells with high metabolic demand (e.g., insulin-producing cells), allow for both intra-device vascularization and a means to spatially distribute the cells within the device to avoid cell death resulting from inadequate nutrient supply where the high density of metabolic demand cannot be satisfied due in large part to diffusion limitations. The scaffold surface and void spaces, or the scaffold material itself, are modified with one or more different adhesion proteins and optionally other biological factors (e.g., anti-inflammatory factors) for enhanced cell adherence and viability.

BRIEF DESCRIPTION OF THE DRAWING

**FIGURE 1: Schematic of scaffold platform and clinical islet transplantation.**

a) Diagram of procedure for islet cell transplantation.
b) Schematic of the multi-functional platform of the microporous scaffold. The scaffold material provides three-dimensional distribution and protection from mechanical stress for cells of multiple sizes. The porosity of the scaffold material is sufficient to permit vascular infiltration. The scaffold material is modified with bioactive factors to enhance adhesion or modulate the surrounding environment, and can serve as a means to locally deliver agents. Further, the scaffold material may serve as a platform for the delivery of adherent cells, which can grow and proliferate on the scaffold.

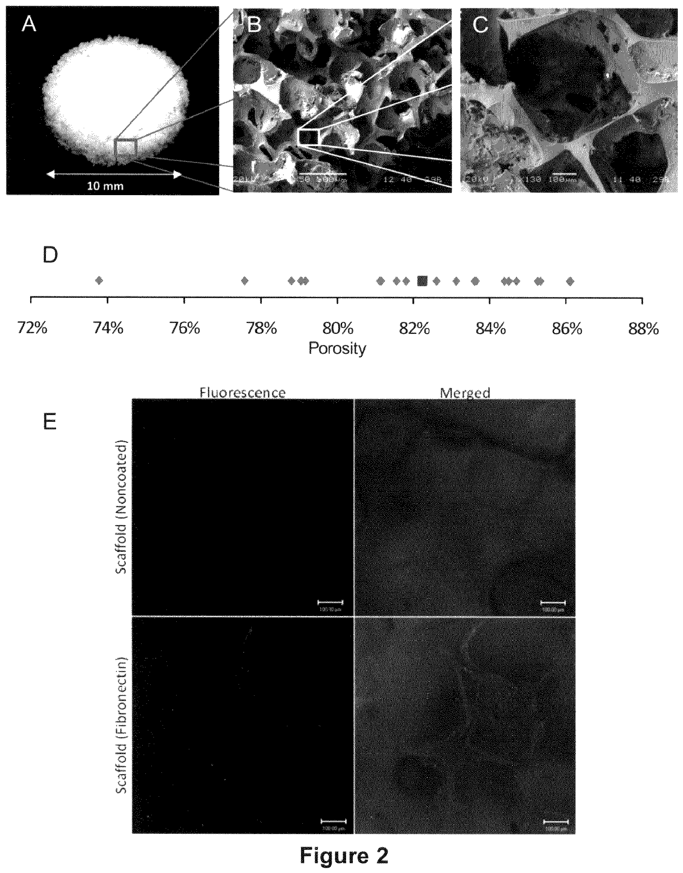

**FIGURE 2: Analysis of scaffold pore structure.**

a) Photographic image of macroporous scaffold structure.
b) Image of macroporous scaffold structure by scanning electron microscopy (JEOL, JSM-5600LV, 29Pa, 20kV).
c) Image of macroporous scaffold structure by scanning electron microscopy at higher magnification (JEOL, JSM-5600LV, 29Pa, 20kV).
d) Distribution graph of scaffold porosity (n=20). The green square indicates average scaffold porosity at 85%.
e) Confocal images (left) and merged confocal and fluorescent images (right) of noncoated scaffold (top) and fibronectin coated scaffold (bottom) stained with anti-fibronectin-biotin primary antibody and streptavidin-FITC secondary antibody (Zeiss LSM510).

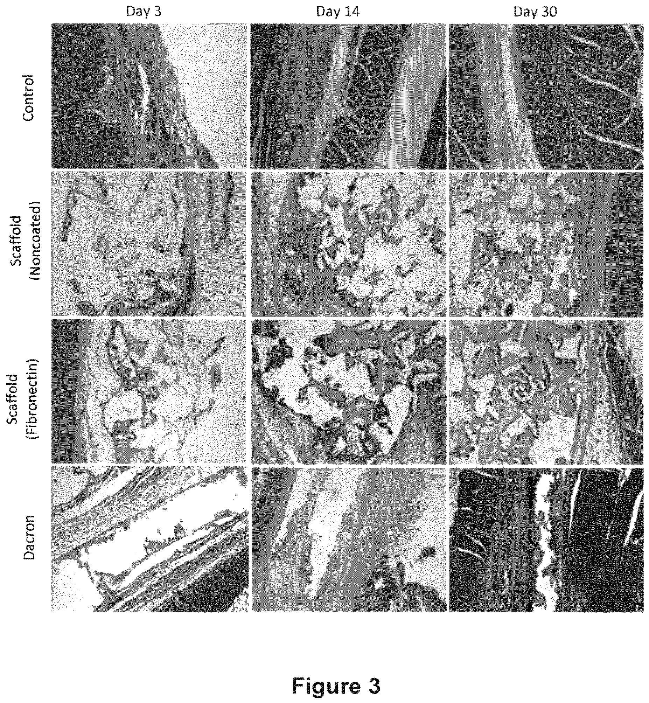

**FIGURE 3: Scaffolds are biostable for up to 30 days after implantation into rats.**

a) Histological cross-sections of scaffolds at 10X magnification stained with Masson's trichrome stain at days 3, 14 and 30 after implantation into 1 cm subcutaneous pockets in male Lewis rats. Top row: control (absence of materials). Second row: noncoated PDMS macroporous scaffolds. Third row: fibronectin-coated PDMS macroporous scaffolds. Bottom row: negative control (Dacron material).

**FIGURE 4: Identification of blood vessel infiltration into scaffolds.**
Histological cross-sections of noncoated and fibronectin coated scaffolds at 40X magnification stained with Masson's trichrome stain at day 14 and day 30 after implantation into 1 cm subcutaneous pockets in male Lewis rats. BV: blood vessels. SS: scaffolds.

**FIGURE 5: Analysis of collagen deposition and total infiltration into scaffolds.**

A) Quantification of collagen deposition into noncoated and fibronectin coated scaffolds via Metamorph Analysis of blue areas in samples stained with Masson's trichrome stain.
B) Quantification of total cell and matrix infiltration into noncoated and fibronectin coated scaffolds via Metamorph Analysis of total area not occupied by scaffold material in samples stained with Masson's trichrome stain.

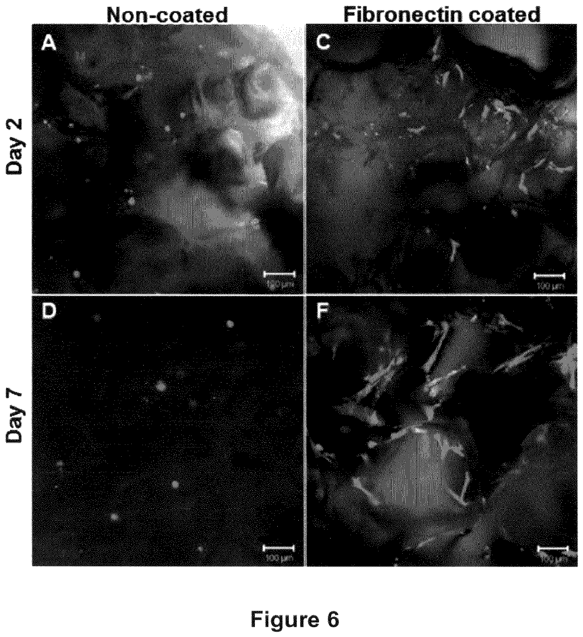

**FIGURE 6: Cellular adhesion and growth on scaffolds.** Visualization of mesenchymal stem cells loaded in noncoated (left) and fibronectin-coated (right) scaffolds and cultured in standard culture conditions (24 well plate, full culture media) for two days (top) and seven days (bottom). Samples were stained with fluorescent live/dead dye (calcein AM (green) and EthD-1 (red)).

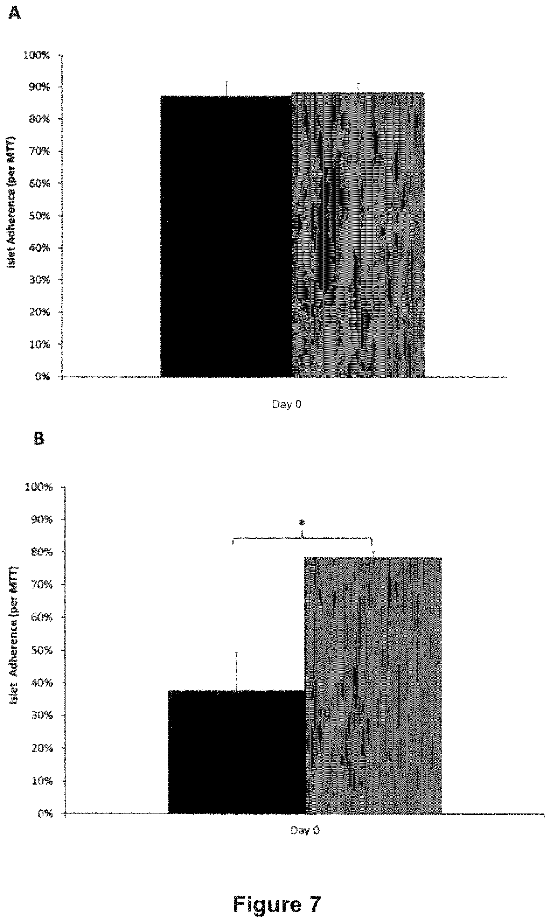

**FIGURE 7: Adherence of islets seeded in *scaffolds.**

A) Graph showing retention of rat islets within scaffold alone (black bar) and scaffold with fibrin (gray bar) as measured by a colorimetric MTT assay at day 0 after loading.

B) Graph showing retention of non-human primate islets within scaffold alone (black bar) and scaffold with fibrin (gray bar) as measured by MTT assay at day 0 after loading.

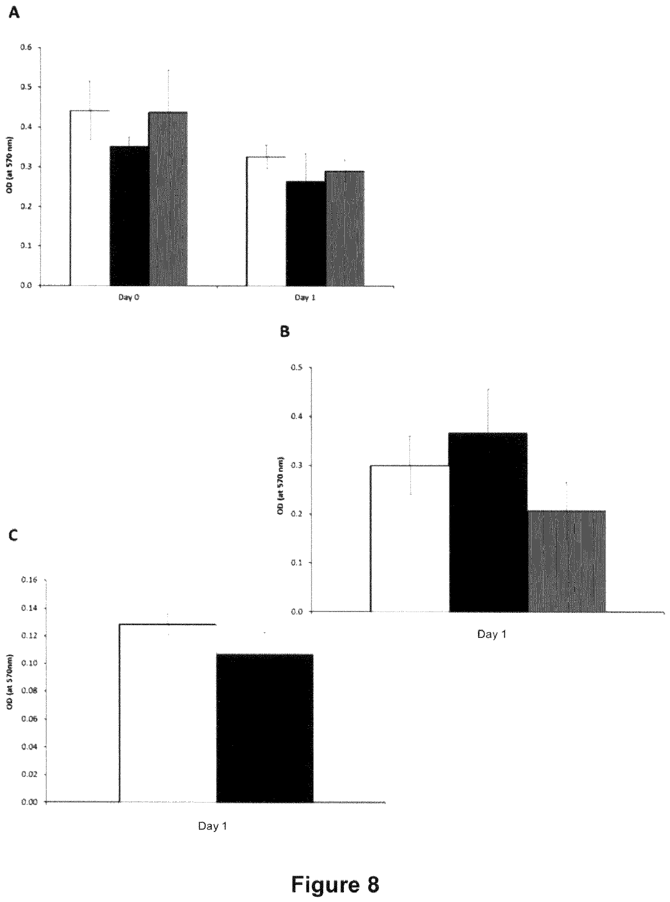

**FIGURE 8: Viability of islets seeded in scaffolds.**

Viability of 1500 IEQ within: a standard two-dimensional culture dish control (white bar); a PDMS macroporous scaffold without fibrin (black bar); or a fibronectin-coated PDMS macroporous scaffold with fibrin (gray bar); as measured by MTT assay. Fibrin was loaded onto the scaffold following islet loading, to fill in the scaffold void space. Islets were cultured for 0 or 24 hours for rat islets and 24 hours for non-human primate and human islets. A: rat islets; B: non-human primate islets; C: human islets.

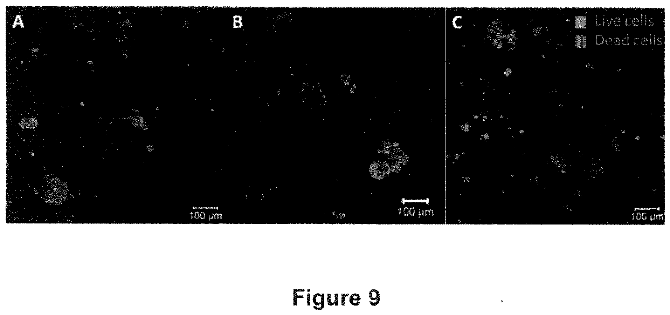

**FIGURE 9: Spatial distribution and viability of islets seeded in scaffolds.**

Confocal microscope images of islets stained with fluorescent live/dead dye (calcein AM (green) and EthD-1 (red)) within PDMS macroporous scaffolds following 24 hours of culture. A: rat islets; B: non-human primate islets; C: human islets.

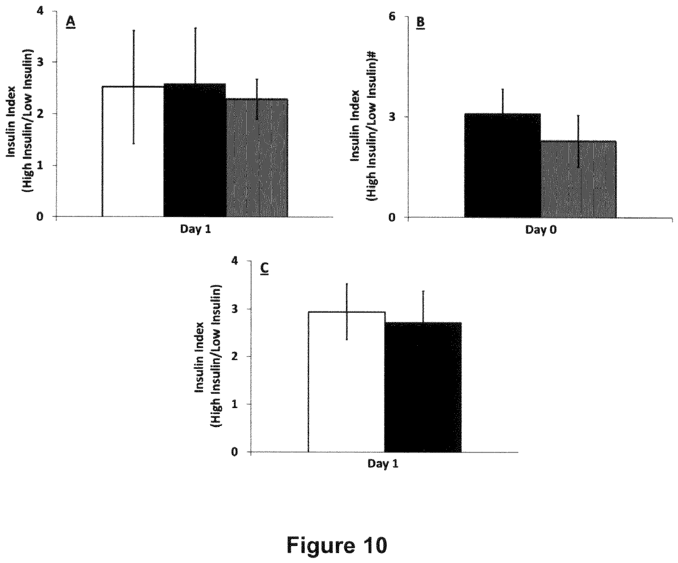

**FIGURE 10: Insulin secretion of islets seeded in scaffolds.**

Insulin ELISA assay showing functional insulin secretion rates after low (40 mg/dL) and high (300 mg/dL) glucose stimuli for 150 IEQ in: a standard two-dimensional culture dish control (white bar); a PDMS macroporous scaffold without fibrin (black bar); or a PDMS macroporous scaffold with fibrin (gray bar). Results are expressed as stimulation index, which is the total insulin output at high glucose divided by the total insulin output at low glucose. Human and rat islets were cultured for 24 hours, while non-human primate islets were evaluated six hours after loading. A: rat islets; B: non-human primate islets; C: human islets.

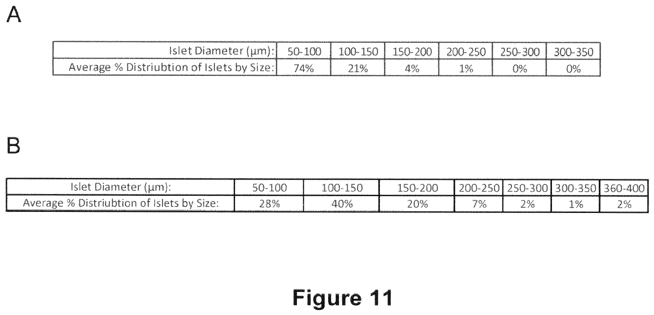

**FIGURE 11: Average diameter of islets retained in scaffolds.**

A) Table showing average diameter of non-human primate islets seeded in scaffolds.

B) Table showing average diameter of human islets seeded in scaffolds.

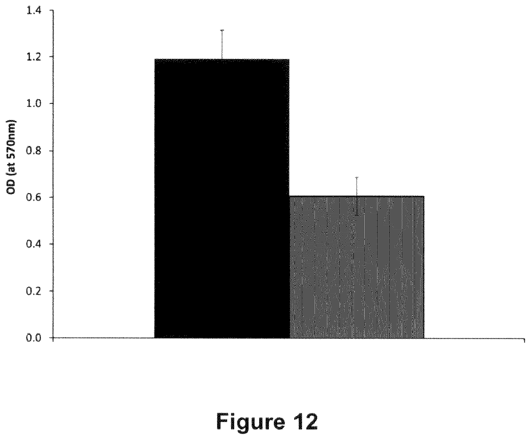

**FIGURE 12: Comparison of islet viability in macroporous and microporous structures.**

Graph showing islet viability of 1000 IEQ cultured for 48 hours at 5% oxygen in a PDMS macroporous scaffold (black bar, left) or a microporous 2% agarose gel (gray bar, right), as measured by MTT assay.

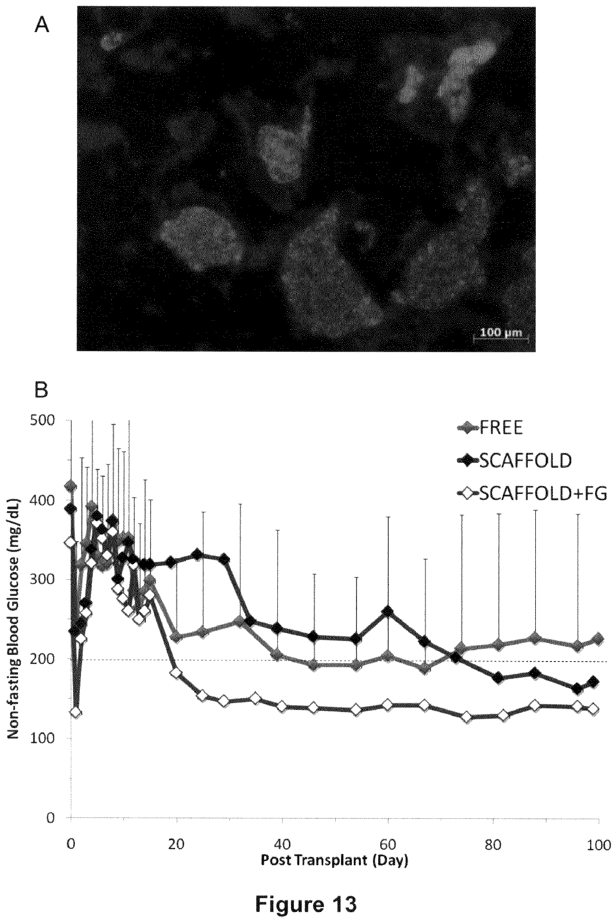

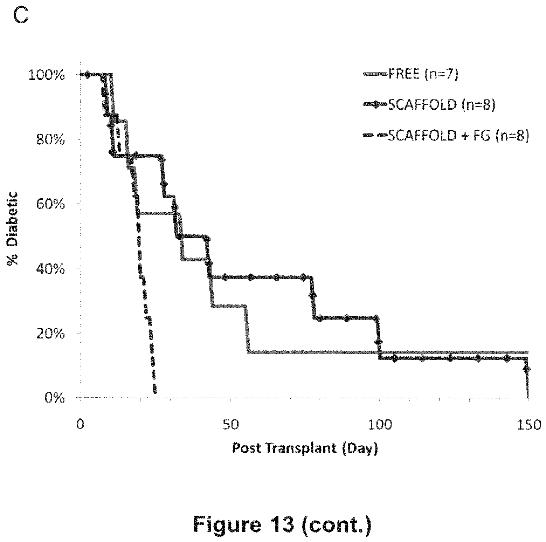

**FIGURE 13: *In vivo* performance of scaffolds for euglycemia restoration in a diabetic mouse model.**

A) Staining of scaffold for islets (red) and nuclei (DAPI) 74 days after implantation into the epididymal fat pad of a diabetic mouse model.

B) Postoperative blood glucose levels of diabetic mice implanted with islets alone (light blue diamonds), islets seeded in a fibronectin-coated scaffold (dark blue line, black diamonds) or islets seeded in a fibronectin-coated scaffold then coated with fibrin/PDGF (white diamonds).

C) Diabetic state graphs for mice with free islets (light blue solid line), islets seeded in a fibronectin-coated scaffold (dark blue beaded line) and islets seeded in a fibronectin-coated scaffold then coated with fibrin/PDGF (dotted line).

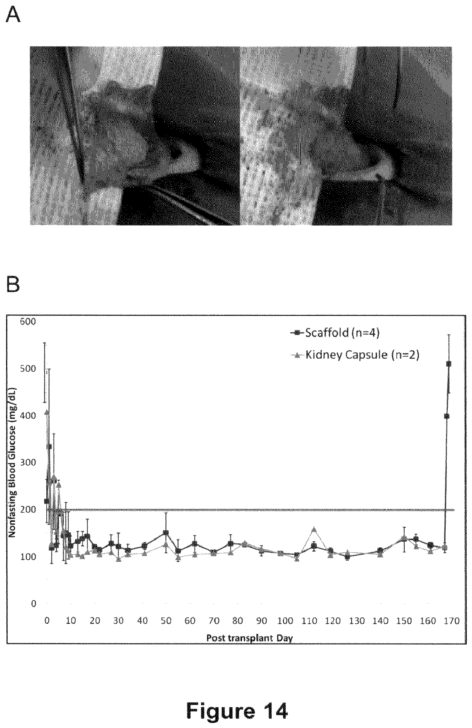

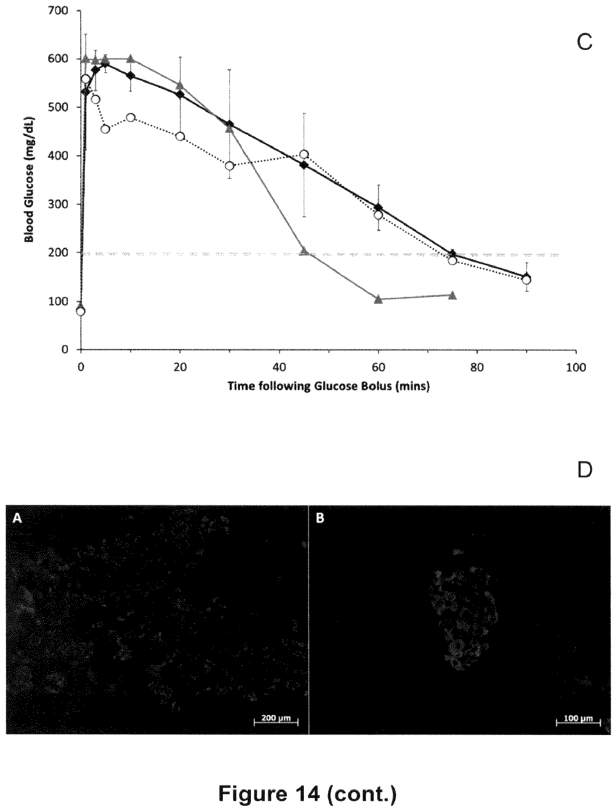

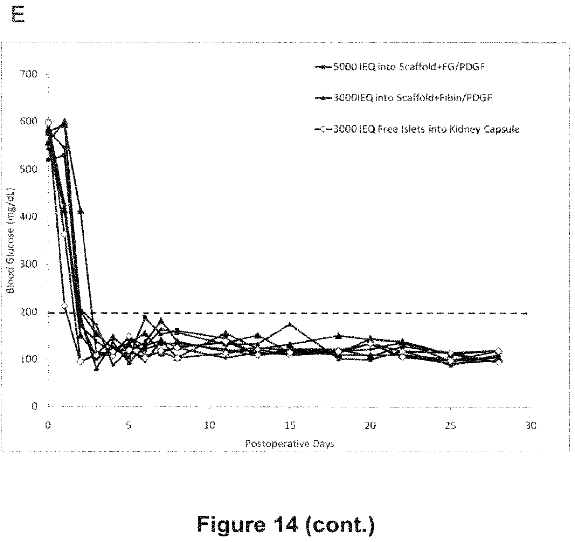

**FIGURE 14: *In vivo* performance of scaffolds in a diabetic rat model.**

A) Photographs of a PDMS macroporous scaffold loaded with islets in a spread omentum (left) and wrapped up in the omentum (right) of a Lewis rat.

B) Graph showing nonfasting blood glucose levels of chemically induced diabetic Lewis rats following the transplantation of 1500 IEQ syngeneic islets in a PDMS macroporous scaffold into the omental pouch site (squares) or as free islets into the standard kidney capsule transplant site (triangles). The graft was removed at 168 days.

C) Graph showing results of intravenous glucose tolerance test performed on functional graft recipients over 100 days post transplant. Black diamond, solid line: islets in silicone scaffold in omentum. Open circle, dashed line: islets freely loaded in omentum. Gray triangle, solid line: islets freely loaded into the kidney capsule.

D) Photographs showing 10X (A) and 20X (B) magnifications of immunofluorescence-stained islets within PDMS macroporous scaffolds following explantation of the grafts (blue: nuclei stained with DAPI; green: islets).

E) Graph showing nonfasting blood glucose levels of chemically induced diabetic Lewis rats following the transplantation of 3000 or 5000 IEQ allogeneic islets in a PDMS macroporous scaffold with fibrin/PDGF into the omental pouch site (3000: triangle: 5000:squares) or as free islets into the standard kidney capsule transplant site (diamonds).

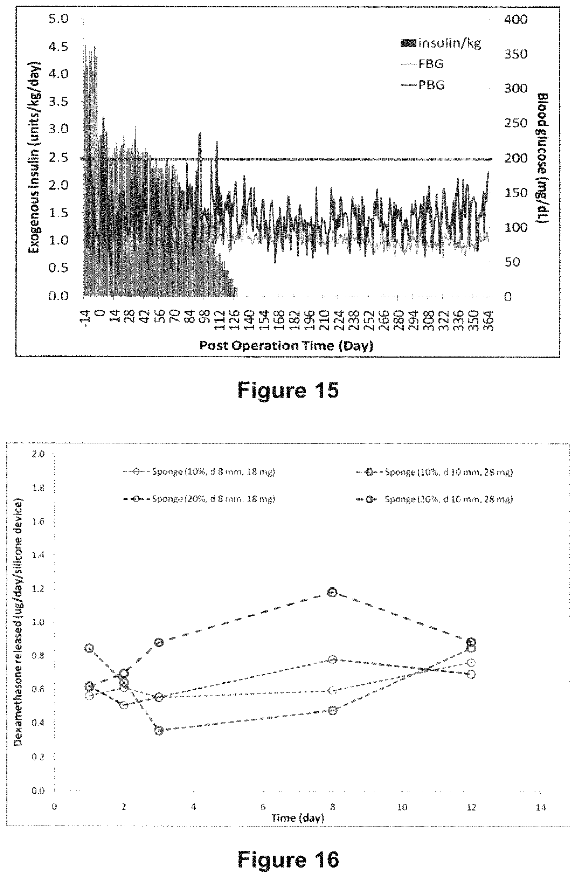

**FIGURE 15: *In vivo* performance of scaffolds in a diabetic baboon model.**
Graph showing fasting blood glucose (FBG; yellow), post-prandial blood glucose (PBG; green), and exogenous insulin levels (insulin/kg; blue) for a baboon receiving 25,000 allogeneic IEQ/kg loaded within 6 PDMS macroporous scaffolds and implanted into the omental pouch. The baboon received intravenous anti-CD154 mono-therapy (20 mg/kg) on post-operative days 0, 1, 4, 10, 18 and 28 and every week thereafter as maintenance.

**FIGURE 16: Elution profiles for dexamethasone incorporated in scaffolds.**
Elution profiles for 10% or 20% w/v dexamethasone incorporated within a PDMS macroporous scaffold when incubated in a buffer solution. Scaffolds of 8 mm and 10 mm in diameter were tested.

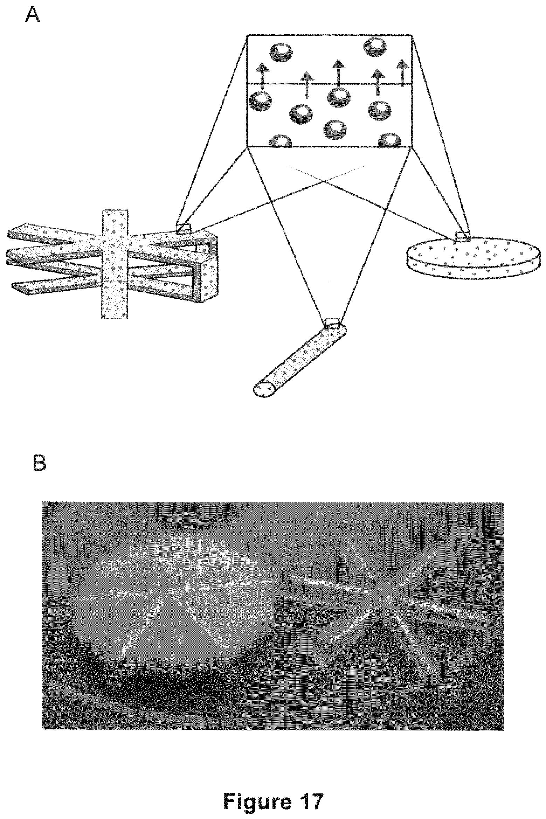

**FIGURE 17: Design of agent-releasing elements.**

A) Examples of potential drug-releasing geometries. Drugs may be incorporated into a biocompatible material such as PDMS and shaped into a disk, rod, or outer cage for implantation with the macroporous scaffold.
B) Photograph of a drug-releasing "cage" design, where the drug-releasing material is fabricated into an outer cage that can house the macroporous scaffold.

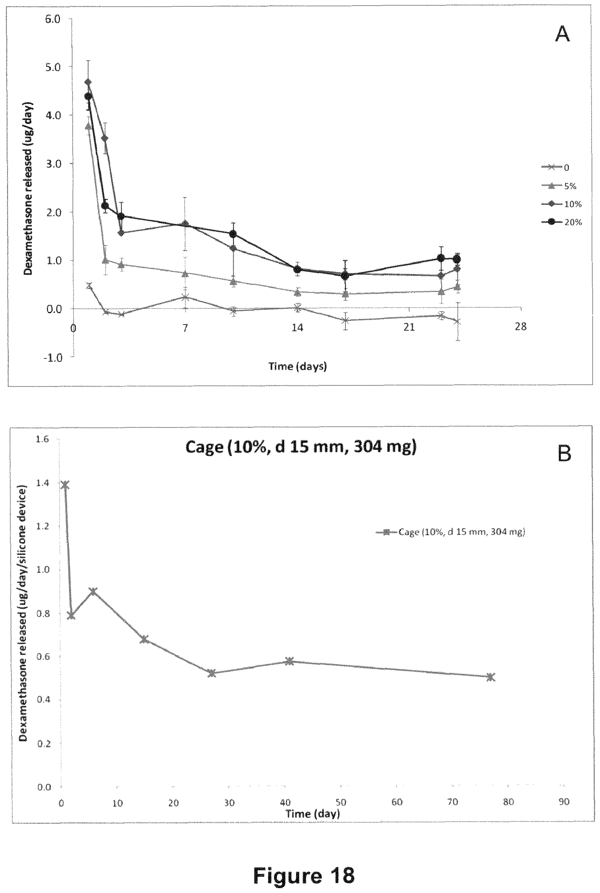

**FIGURE 18: Elution profiles for the release of dexamethasone incorporated into agent-releasing elements.**

A) Elution profiles for the release of dexamethasone incorporated at 0%, 5%, 10% or 20% w/v into PDMS disks.
B) Elution profile for the release of dexamethasone incorporated at 10% w/v into a PDMS cage.

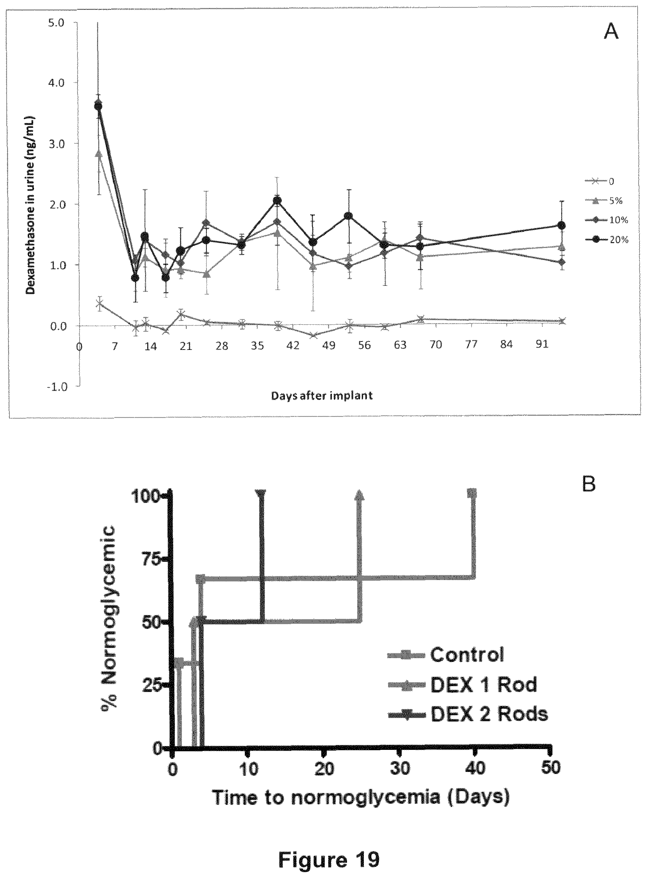

**FIGURE 19: *In vivo* performance of agent-releasing elements in a diabetic mouse model.**

a) Urine measurements of dexamethasone level in mice with a subcutaneous implant of a dexamethasone releasing PDMS disk (with dexamethasone incorporated at 0%, 5%, 10%, or 20% w/v) as a function of days after implant.
b) Graph illustrating the time to reversal to normoglycemia for chemically induced diabetic mice with syngeneic islet transplants within the scaffold at the epididymal fat pad site, with: no dexamethasone releasing rod surrounding the scaffold (squares), one dexamethasone releasing rod surrounding the scaffold (triangles) or two dexamethasone rods surrounding the scaffold (upside-down triangles).

<u>DETAILED DESCRIPTION OF THE INTENTION</u>

The invention relates to biocompatible scaffolds with a controllable pore size range. In some embodiments, the pore size range is 25-650 $\mu$m. In certain embodiments, the pore size range is 250-425 $\mu$m, which is conducive to housing, e.g., typical 150 $\mu$m diameter pancreatic islets. In other embodiments, some or all of the pore sizes are smaller (e.g., 225 $\mu$m, 200 $\mu$m, 175 $\mu$m, 150 $\mu$m, 100 $\mu$m, or less), for housing smaller cells (e.g., smaller islets, or individual cells or aggregates of individual cells such as beta cells and other therapeutic agent-releasing cells). The pore sizes may in certain embodiments be larger, e.g., for housing larger cells or tissue samples. The pores may also be fabricated with a mixture of pore sizes. In certain embodiments, the scaffold has pore sizes ranging from 50-500 $\mu$m, 60-450 $\mu$im or 75-400 $\mu$m. Pore sizes may be randomly distributed or may be fabricated to form patterns. For example, the pores may be positionally asymmetric. In some embodiments, the pores may be fabricated with larger pores in one part of the scaffold and smaller pores in another part of the scaffold. In certain embodiments, the pores may be fabricated with larger pores on one side of the scaffold (e.g., the top) and smaller pores on another side (e.g., the bottom), creating a gradient of pore sizes. In some embodiments, the pores are fabricated to provide substantially uniform vascularization throughout the scaffold. The scaffolds may be fully or partially macroporous. The scaffolds have high biocompatibility after extended implantation and are suitable for *in vivo* applications. In some embodiments, the viability and functionality of cells (e.g., insulin-producing cells) are not adversely affected by loading within the scaffolds. In some embodiments, cells (e.g., insulin-producing cells) and/or cell aggregates distribute evenly throughout the scaffold.

In certain embodiments, the scaffolds are fabricated using the solvent casting and particulate leaching technique (SCPL). The scaffolds are fabricated from organosilicone. Silicone molds can be created by combining varying ratios of particulates and silicone polymer. In some embodiments, the ratio is 50-90% v/v. In some embodiments, the ratio is 50%, 55%, 60%, 65%, 70%, 75%, 80%, 85%, 86%, 87%, 88%, 89%, 90%, 91%, 92%, 93%, 94%, 95%, 96%, 97%, 98%, or 99% v/v particulates to silicone polymer. In one embodiment, the ratio of particulates to silicone polymer is 90% v/v. In some embodiments, the particulates are salt crystals, gelatin spheres, paraffin spheres, sodium tartrate, sodium citrate and/or glucose grains. In certain embodiments, the particulates are sodium chloride. In certain embodiments, the silicone polymer is the organosilicone polymer polydimethylsiloxane (PDMS). Pore size and degree of porosity can be individually controlled by varying the particle size and polymer to particle ratio, respectively, using methods known in the art. The molds in which the particulate/silicone mixture is loaded may vary in dimension depending on the type of cells to be transplanted and the site of implantation. In some embodiments, the scaffold has a porosity of 20-99%, 30-99%, 40-98%, 50-97%, 60-96%, 70-95%, 75-90%, or 80-90%. In some embodiments, the porosity of the scaffold is greater than 20%, 30%, 40%, 50%, 60%, 75%, 80%, 81%, 82%, 83%, 84%, 85%, 86%, 87%, 88%, 89%, 90%, 91%, 92%, 93%, 94%, 95%, 96%, 97%, 98% or 99%.

The scaffolds may be of any desired shape. The shape of the scaffolds may vary based on the types of cells to be implanted, the intended therapeutic effect, and/or the location of the implant, for example. The skilled practitioner can assess the shape(s) preferred for the intended application(s).

The scaffold surface is coated with fibronectin, and fibrin glue and PDGF are loaded into the void spaces, either individually or in various combinations.

In some embodiments, the scaffold has the capacity to be stable at greater than 50%, 60%, 70%. 75%, 80%, 85%, 90%, or 95% void space. In certain embodiments, the scaffold has the capacity to be stable at greater than 80% void space.

In certain embodiments, the invention relates to biocompatible, highly porous organosilicone scaffolds with controllable pore size and porosity that can be fabricated and used for transplantation of insulin-producing cells. The scaffolds may be used for transplanting the cells into alternative, non-hepatic sites by addressing the issues of spatial distribution of insulin-producing cells, mechanical protection, and infra-device vascularization.

Besides pancreatic islets, which are considered one preferred cell/tissue type for regulating sugar and energy metabolism, and for treating diabetes, the scaffolds of the invention and methods involving those scaffolds may also be used for other cell therapy model systems. Cells for implantation may delivery a therapeutic benefit, e.g. by expressing a therapeutic factor *in vivo*. Examples of such cells include, but are not limited to, cells that produce: dopamine to treat Parkinson's disease (Minquez-Castellanos et al., J Neurol Neurosurg Psychiatry in press (2007)); growth hormone to treat dwarfism (Chang et al., Trends Biotechnol 17:78-83 (1999)); factor VIII and factor IX (Chang et al., Trends Biotechnol 17, 78-83 (1999)) to treat hemophilia; and erythropoietin to treat anemia (Rinsch et al., Kidney Intern 62:1395-1401 (2002)). Many more beneficial cell produced factors or cellular/tissue activities may be imagined. The implanted cells may express and/or deliver more than one therapeutic factor, or may comprise two or more cell types delivering one or more therapeutic factors. The implanter cells may also or alternatively express and/or deliver an agonist, analog, derivative, fusion, or fragment of a therapeutic factor to deliver a therapeutic effect. **Implanted non-human cells may also express and/or deliver a chimera.**

The implanted cells may also or alternatively deliver a therapeutic effect without secreting a diffusible factor, e.g., by providing an enzymatic activity that, for example, converts a substrate into a product having a beneficial effect, and/or metabolizing, sequestering, or absorbing a detrimental substance. The implanted cells may deliver a therapeutic effect through a biological material-linked factor, such as a cell surface-linked factor..

The cells may naturally deliver a therapeutic effect, without genetic modifications, or may be genetically engineered to do so. For example, the cells may be transfected with expression vectors that express one or more therapeutic and/or helper cell factors. In another embodiment, the cells may comprise, consist of, or consist essentially of cells transfected with expression vectors that express one or more therapeutic and/or helper cell factors. Such expression may be in a constitutive or in a regulated manner, e.g., in response to biological modulators in the bloodstream or tissues to which the scaffold is exposed. These and other expression systems and methods for making them are well known to the skilled practitioner.

The cells may be, for example, autologous, heterologous, syngeneic, allogeneic, or xenogeneic cells. The cells may be derived from cadaver tissue or from living tissue. The cells may be of non-mammalian or mammalian origin, non-human origin or human origin, self or non-self. The cells may be **pluripotent, multipotent, non-human totipotent, or differentiated embryonic or adult stem** cells; primary differentiated cells; or immortalized cells, among other cell types. Stem cells may comprise, e.g., cells derived from cord blood, amniotic fluid, menstrual blood, placenta, Wharton's jelly, cytotropoblasts, and the like. The cells may also comprise any combination of the above-listed cell types.

The cells that provide a therapeutic effect may be implanted alone or in combination with other cell types (e.g., Sertoli cells, mesenchymal and bone marrow derived cells, endothelial progenitor cells, stem cells, regulatory T cells $T_{reg}$, etc., each referred to genetically as implant "helper cells") that provide growth factors and/or other beneficial agents for establishment, maintenance or expansion of the implanted cells, or otherwise to help the implanted cells deliver a therapeutic effect. In one embodiment, the cells that provide a therapeutic effect are implanted with mesenchymal stem cells (MSCs).

The scaffolds and methods of this invention may be used to treat disorders including, but not limited to: diabetes, Parkinson's disease, anemia, dwarfism, hemophilia, amyloidosis, immune system disorders, inflammations, chronic pain, arthritis, hypertension, disorders of the nervous system, metabolic disorders, endocrine disorders, lymphoproliferative disorders, myeloproliferative disorders, myelodysplastic syndromes, stem cell disorders, phagocyte disorders, histiocytic disorders, abnormalities of erythrocytes or platelets, plasma cell disorders, acute leukemias, chronic leukemias, malignancies (breast carcinoma, Ewing Sarcoma, neuroblastoma, renal cell carcinoma, etc.), hypothyroidism, hypopituitarism, hypogonadism, graft failure, graft versus host disease (GVD), veno-occlusive disease, side effects from pre-transplant chemotherapy (such as excessive bleeding, infertility, and renal as well as lung and heart complications), and other disorders and diseases that will be recognized by the skilled practitioner.

Exemplary therapeutic factors which may be delivered by the transplanted cells include, but are not limited to, one or more of: insulin, glucagon, erythropoietin; Factor VIII; Factor IX; hemoglobin; albumin; neurotransmitters such as dopamine, gamma-aminobutyric acid (GABA), glutamic acid, serotonin, norepinephrine, epinephrine, and acetylcholine; growth factors such as nerve growth factor (NGF), brain-derived neurotrophic factor (BDNF), neurotrophin-3 (NT-3), neurotrophin 4/5 (NT-4/5), ciliary neurotrophic factor (CNTF), glial cell line-derived neurotrophic factor (GDNF), cholinergic differentiation factor/leukemia inhibitory factor (CDF/LIF), epidermal growth factor (EGF), insulin-like growth factor (IGF), fibroblast growth factor (FGF), and platelet-derived growth factor (PDGF); pain inhibitors such as Substance P, catecholamines, dynorphins, endorphins, or enkephalins; hormones such as parathyroid hormone or growth hormone; immunomodulators such as granulocyte-macrophage colony stimulating factor (GM-CSF); neuromodulators; lymphokines; cytokines; cofactors; antibodies; aptamers; and enzymes. Choice of one or more therapeutic factors and the concentrations at which they are produced and released from the cells and thereby from the scaffolds to the subject are dictated by the needs of the subject (e.g., patient) being treated, the selected location of implantation and other factors that may be readily determined empirically by the skilled practitioner.

In some embodiments, the therapeutic factor has insulin-like or insulin-regulatory activity. In certain embodiments, the therapeutic factor is insulin or an insulin analog. In certain embodiments, the therapeutic factor is a precursor form of insulin, such as preproinsulin or proinsulin. In certain embodiments, the therapeutic factor is an insulin chimeric or fusion protein.

In some embodiments, the therapeutic factor(s) are released from the implanted cells due to the receipt of a stimulus or signal from the host (e.g., changes in blood levels of glucose, hormones, metabolic signaling agents, chemical signaling molecules, etc.).

In some embodiments, the therapeutic effect comprises regulation of insulin levels in the blood. In certain embodiments, the therapeutic effect comprises regulation of glucose levels in the blood. In other embodiments, the therapeutic effect comprises regulation of levels of one or more other biological response regulators in the blood of the subject.

In some embodiments, the scaffolds also contain agents that aid long term survival and function of the transplanted cells. Such agents include, e.g., agents for vascularization (e.g., VEGF), anti-inflammatory agents (e.g., anti-TNF alpha, lisofylline, pentoxifilline, alpha 1-antitrypsin, interleukin-1 (IL-1), interleukin-10 (IL-10), interleukin-1 receptor antagonist peptide (IRAP), TGF-beta, antibodies to IL-1 , interferon gamma, TNF-alpha, anti-tissue factor, complement inhibitors, COX-2 inhibitors, calcineurin inhibitors (e.g., cyclosporine, tacrolimus, etc.), glucocorticoids (e.g., dexamethasone, cortisol, prednisolone, loteprednol etabonate, flucinolone acetonide, etc.), and inhibitors of lymphocyte trafficking (e.g. fingolimod, etc.)); oxygen generating, releasing or transport-enhancing agents (e.g., encapsulated peroxides or perfluorocarbons (PFCs)); cytoprotective/antiapoptotic agents/molecules, tolerance-inducing molecules (e.g., the Power-Mix described in Zheng et al., immunity 19(4):503-514 (2003), or wherein said Power-Mix comprises (1) an agonist to IL-2, immunoglobulin, and/or a fusion protein; (2) antagonist-type IL-15-related cytolytic immunoglobulin and/or a fusion protein; and (3) plus or minus rapamycin); IL-10 and IL-10 fusions; costimulatory blocking agents including antibodies, fusion proteins, small molecules, galectin-1, aptamers, antibodies and aptamers to lymphocyte activation markers (e.g., 4BB1); adhesion molecules (e.g., CD103, etc.) and other molecules involved in the delivery of signals to lymphocytes (e.g., LFA1, LFA3, 4BB1, and CD45, etc.); EBNA-like molecules; IL-35-, IL12-, and IL12-receptor-targeting antibodies and aptamers; anti-IL-17 antibodies; anti-IL-17 receptor antibodies and aptamers; and anti-IL-6 antibodies and IL-6 receptor antibodies and aptamers; etc.); immunosuppressive agents (e.g., oATP, calcineurin inhibitors (e.g., cyclosporine, tacrolimus, etc.), protein kinase C inhibitors (e.g., AEB071, etc.), inhibitors of proliferation signals (e.g., sirolimus, everolimus, JAK3 inhibitors, etc.), inhibitors of nucleotide synthesis (e.g., azathioprine, mycophenolic acid MPA / mycophenolate mofetil MMF, leflunomide, FK778, etc.), glucocorticoids (e.g., dexamethasone, cortisol, prednisolone, loteprednol etabonate, flucinolone acetonide, etc.), inhibitors of lymphocyte trafficking (such as fingolimod and other sphingosine-1-phosphate receptor 1 modulators, etc.); inhibitors of cell surface receptor activation (such as depleting or nondepleting antibodies and fusion proteins including but not limited to Thymoglobulin ATG, muromonab-CD3, alemtuzumab, rituximab, daclizumab, basiliximab, belatacept, campath-1H, Prograf, anti IL-2r, MMF, FTY, LEA, and others, etc.); oxygen generating, releasing or transport-enhancing products; and growth factors (e.g., IGF-I, IGF-II, INGAP, exendin-4, GLP-1, HGF, etc.). In certain embodiments, some or all of the agents are released with slow/sustained release characteristics, e.g., in slow/sustained release cartridges, coatings, encapsulations, micro- or nanospheres, etc.

In certain embodiments, some or all of the agents exhibit slow/sustained release for over at least 3, 10, 12, 20, 30, 40, 50, 60, 70, 80, 90, 100, 200, 300, 365, 500 or 730 days, or any other period adequate for the desired purpose. In one embodiment, the scaffold comprises slow-release dexamethasone. In one embodiment, the scaffold comprises slow-release fingolimod (FTY720).

In some embodiments, an agent is released from the scaffold. The agent may be incorporated into, e.g., a 3-D formulation. In some embodiments, some or all of the agent is released with slow/sustained release characteristics. In certain embodiments, the agent is an anti-inflammatory or immunosuppressive agent, e.g., anti-TNF alpha, lisofylline, pentoxifilline, alpha 1-antitrypsin, interleukin-1 (IL-1), interleukin-10 (IL-10), interleukin-1 receptor antagonist peptide (IRAP), TGF-beta, antibodies to IL-1, interferon gamma, TNF-alpha, anti-tissue factor, complement inhibitors, COX-2 inhibitors, calcineurin inhibitors (e.g., cyclosporine, tacrolimus, etc.), glucocorticoids (e.g., dexamethasone, cortisol, prednisolone, loteprednol etabonate, flucinolone acetonide, etc.), inhibitor of lymphocyte trafficking (e.g. fingolimod, etc.), protein kinase C inhibitors, inhibitors of proliferation signals (e.g., sirolimus, everolimus, JAK3 inhibitors, etc.), inhibitors of nucleotide synthesis (e.g., azathioprine, mycophenolic acid MPA / mycophenolate mofetil MMF, leflunomide, etc.), and inhibitors of cell surface receptor activation (e.g., depleting or nondepleting antibodies and fusion proteins including but not limited to Thymoglobulin ATG, muromonab-CD3, alemtuzumab, rituximab, daclizumab, basiliximab, belatacept, campath-1H, etc.). In certain embodiments, the agent is a hydrophobic agent. In some embodiments, the agent comprises dexamethasone, prednisolone, cyclosporine, tacrolimus, sirolimus, everolimus, fingolimod (FTY720), mycophenolic acid, etc. Organosilicone materials such as PDMS are particularly suitable for the preparation of sustained release formulations of hydrophobic drugs (Malcolm, K. et al. J. Contr. Rel. 2003, 90, 217). However, any material that may be used to prepare sustained release formulations of hydrophobic drugs is suitable for and encompassed by the present invention.

In some embodiments, the scaffold acts as a membrane enclosing an interior reservoir system with the agent(s) to be released. In some embodiments, the agent is incorporated into the scaffold structure itself. In certain embodiments, the scaffold forms a non-erodible matrix system with the agent, wherein the agent is dispersed in the polymer. A number of siloxane-based implants for sustained release progestin delivery (6 months to 7 years) are commercially available (Croxatto, H. B. Contraception 2002, 65, 15) proving the feasibility and safety of this approach. In some embodiments, the material of the scaffolds also serves as the polymeric matrix material for the sustained release of the therapeutic agent.

Alternatively, or additionally, the agent may be incorporated into an element that is not connected to the part of the scaffold containing the implanted cells, which is then placed within, surrounding or adjacent to the scaffold structure upon implant. In some embodiments, the separate element is nonporous. The separate element may have any shape capable of effectively releasing an agent for the purposes of the invention. In some embodiments, the separate element is shaped into a disk, rod or outer cage. In certain embodiments, the separate element is an outer cage that surrounds the scaffold. In certain embodiments, the separate element is designed for periodic replacement, and provides sustained release of the incorporated agent for arbitrary prolonged periods as desirable. The incorporated agent may be released for periods of, e.g., hours, days, weeks, or years before replacement. The separate element may be made of, e.g., PDMS and/or any other biocompatible materials capable of being fabricated with and releasing agents. In some embodiments, the separate element is fabricated from the same material as the scaffold. In some embodiments, the separate element releases the same agent as the scaffold.

In one embodiment, anti-inflammatory and/or immunosuppressive molecules are tethered to or incorporated into the scaffold to reduce the host inflammatory response to the implant. Exemplary anti-inflammatory/ immunosuppressive agents include, e.g., anti-TNF alpha, lisofylline, pentoxifilline, alpha 1-antitrypsin, interleukin-1 (IL-1), interleukin-10 (IL-10), interleukin-1 receptor antagonist peptide (IRAP), TGF-beta, antibodies to IL-1, interferon gamma, TNF-alpha, anti-tissue factor, complement inhibitors, COX-2 inhibitors, calcineurin inhibitors (e.g., cyclosporine, tacrolimus, etc.), glucocorticoids (e.g., dexamethasone, cortisol, prednisolone, loteprednol etabonate, flucinolone acetonide, etc.), inhibitor of lymphocyte trafficking (e.g. fingolimod, etc.), protein kinase C inhibitors, inhibitors of proliferation signals (e.g., sirolimus, everolimus, JAK3 inhibitors, etc.), inhibitors of nucleotide synthesis (e.g., azathioprine, mycophenolic acid MPA / mycophenolate mofetil MMF, leflunomide, etc.), and inhibitors of cell surface receptor activation (e.g., depleting or nondepleting antibodies and fusion proteins including but not limited to Thymoglobulin ATG, muromonab-CD3, alemtuzumab, rituximab, daclizumab, basiliximab, belatacept, campath-1H, etc.) In one embodiment, extracellular matrix (ECM) molecules such as collagen type I or IV, laminin, fibronectin, fibrin, or arginine-glycine-aspartate peptides are incorporated on the surface of the scaffold (Beck et al., Tissue Eng 13(3):1-11 (2007)). In addition to surface modification, the void space is filled (partially or completely, prior/during/after cell loading) with a fibrin matrix. The matrix comprises a growth factor, PDGF. The agents may be directly tethered to the matrix or simply mixed within the materials. In certain embodiments, the agents may be coated or encapsulated for slow release properties. The scaffold incorporates fibronectin on the scaffold surface, and fibrin and PDGF within the void space, individually or in various combinations. In one embodiment, the scaffold material incorporates dexamethasone and/or fingolimod (FTY720).

The scaffolds of the invention may be implanted in any appropriate place within the subject. In certain embodiments, the implant location may be, for example, intraomental (in an omental pouch), subcutaneous, intraperitoneal, intramuscular, or renal subcapsular. In one embodiment, the implant location is subcutaneous.

The scaffolds of the invention may be implanted into any animal host or subject. In some embodiments, the host or subject is a mammal. In a certain embodiment, the host or subject is human.

In any of the above embodiments, the scaffold may further comprise a tether to facilitate manipulation and/or retrieval of the scaffold from a subject.

EXAMPLES

Example 1: Fabrication of Macroporous Silicone Scaffolds

Macroporous silicone scaffolds were fabricated using the solvent casting and particulate leaching technique (SCPL). The silicone polymer was prepared by mixing PDMS monomer with platinum catalyst, 4:1 v/v. The silicone molds were created by combining varying ratios (50-90% v/v) of sodium chloride crystals (Mallinckrodt Baker, NJ) (250 to 425 $\mu$m diameter) and silicone polymer solution. The salt/silicone mixture was loaded into prefabricated, stainless steel molds (10 mm diameter, 2 mm height), pressurized to 1500 psi and incubated at 37°C for 48 hrs to complete silicone cross-linking. The NaCl was then leached out from the scaffolds for at least 72 hrs. Pore size and degree of porosity were individually controlled by varying the particle size and polymer to particle ratio, respectively. For enhanced cell adhesion, the scaffold surface was modified by incubating overnight with fibronectin at 250 $\mu$g/mL

Example 2: Characterization of microporous silicone scaffolds

The macroporous structure of the scaffold was visualized photographically (Fig. 2A) and by scanning electron microscopy (SEM) (Fig. 2B, C). As seen in the SEM images, the scaffold is highly porous and the pore size is representative of the salt crystal diameter. Moreover, the pores are interconnected and tortuous. Final porosity was determined using gross measurements and weights (dry and wet), and calculated with the following formula:

$$porosity = \frac{m_w / \rho_w}{m_w / \rho_w + m_{silicone} / \rho_{silicone}}$$

Porosity of the scaffolds manufactured with 90% w/v sodium chloride crystals was determined to be 85% $\pm$ 5% w/v (Fig. 2D).

The protein-modified scaffold surface was stained with anti-fibronectin-biotin primary and streptavidin-FITC secondary antibodies, and visualized through confocal imaging. The fluorescence imaging demonstrated a homogenously modified scaffold surface with protein coating (Fig. 2E).

We performed *in vivo* studies to assess biocompatibility and stability of the silicone scaffolds, as well as vascular infiltration. *In vivo* biocompatibility was determined for noncoated and protein coated scaffolds by implanting the scaffolds into 1 cm subcutaneous pockets in male Lewis rats and performing histological analyses (H/E) at 3, 14, and 30 days (Fig. 3). Dacron material and absence of a material were also examined as controls.

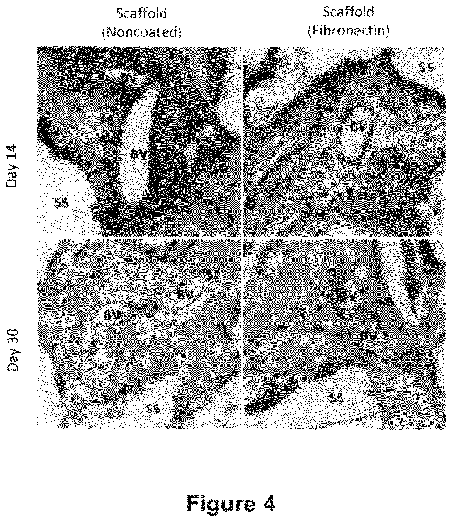

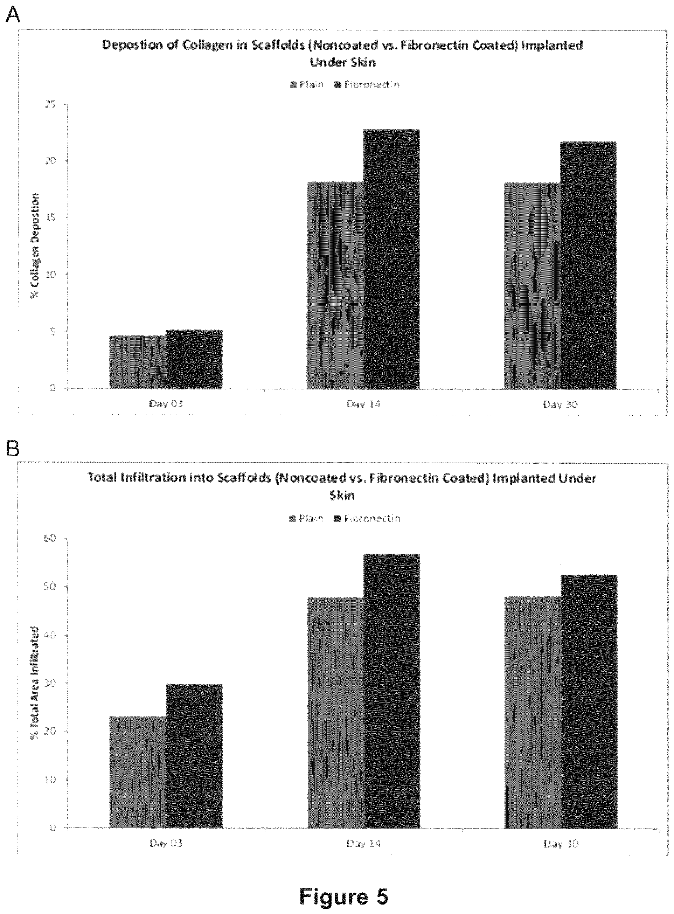

Histological cross-sections showed the biocompatibility of the silicone scaffolds (with or without coating), with blood vessel formation around the scaffold (Fig. 4), significant deposition of collagen and no fibrotic tissue observed (Figs. 4 and 5A). The scaffolds were biocompatible and biostable for up to 180 days. Scaffolds coated with fibronectin encouraged a higher degree of collagen deposition (Fig. 5A) and a higher degree of cellular infiltration and ECM deposition compared to noncoated scaffolds, thereby demonstrating an enhanced integration of the biomaterial within the host (Fig. 5B).

The fibronectin-coated macroporous silicone scaffolds were shown to support greater cellular adhesion and growth of mesenchymal stem cells (MSCs) than noncoated macroporous silicone scaffolds. MSCs cultured on the coated scaffolds for seven days exhibited their standard expanded phenotype (Fig. 6).

Example 3: Islet viability and function in microporous silicone scaffolds

Pancreatic islets from male Lewis rats, non-human primate (NHP) baboons, and human sources were used in experiments to measure islet viability and function in macroporous silicone scaffolds. Islets were loaded into the scaffolds at the desired islet equivalent (IEQ) density by suspending them in a small volume, pipetting them onto the scaffolds and applying a light pressure gradient to distribute the islets into micro-sized pores. Fibrin glue was added to select groups to evaluate islet retention. The islets were cultured in tissue culture dishes at 20% oxygen for up to 24 hours. Two-dimensional cultures were used as controls.

Scaffolds seeded with rat, non-human primate and human islets were inspected for islet spatial distribution and viability by fluorescent live/dead dye staining (calcein AM and EthD-1) and confocal microscopy (Fig. 9). The adherence of the rat and non-human primate islets, and the viability of the rat, non-human primate and human islets, were quantified by MTT assay (Promega, WS), calibrated to cell number via standard curve, for 1500 IEQ per scaffold (Figs. 7 and 8). Functional insulin secretion rates were determined by collecting insulin samples from low (40 mg/dL) and high (300 mg/dL) glucose stimuli for 150 IEQ per scaffold and quantifying by insulin ELISA assay (Fig. 10). The average diameters of the non-human primate islets and the human islets are indicated (Fig. 11), demonstrating that islet retention is dependent on islet size (>35% non-human primate (26% islets $\geq$ 100 $\mu$m); 80% human (71% islets $\geq$ 100 $\mu$m)).

Rat islets within the scaffold (with or without fibrin) performed similarly to controls (p>0.05) for all groups and all assays for the same given day. Non-human primate islets within the scaffold (with or without fibrin) demonstrated similar viability and insulin secretion rate to controls (p>0.05) for all groups. Human islets within the scaffold (without fibrin) performed similarly to controls (p>0.05) for all groups and all assays. Islets within scaffolds with fibrin showed a statistically significant increase in adherence in comparison to islets within scaffolds without fibrin.

To compare islet viability in macroporous and microporous scaffolds, 1000 IEQ islets were loaded into macroporous PDMS scaffolds with fibrin, using a gentle pressure gradient; and into microporous scaffolds by uniformly mixing the islets with 2% liquid agarose, pouring into molds of the same dimensions as the PDMS scaffolds, and allowing to cool to room temperature. The islets were then cultured for 48 hours at 5% oxygen. As determined by MTT assay, viability was enhanced in macroporous scaffolds in comparison with microporous scaffolds (Fig. 12).

These studies indicate that macroporous silicone scaffolds exhibit improved retention of small islets when coated with fibronectin. Further, the macroporous silicone scaffolds retain similar islet viability compared to controls (p>0.05), as well as high functional indices, with no inhibition of islet function as compared to controls.

Example 4: *In vivo* performance of macroporous silicone scaffolds

To assess the *in vivo* efficacy of the scaffolds in restoring euglycemia in a syngeneic streptozotocin (STZ)-diabetic mouse model, fibronectin-coated scaffolds with or without fibrin and PDGF were loaded with 500 IEQ mouse C57BL/6J islets and implanted into the epididymal fat pad (EFP) of STZ-diabetic mice. Scaffolds were folded into the center of the EFP and secured. The control group received free islets into an EFP pocket. On day 74, the scaffold implant with fibrin and PDGF was stained for islets (red) and nuclei (DAPI) (Fig. 13A). The scaffolds with fibrin and PDGF restored normoglycemia (defined as consistent nonfasting glycemia levels < 200 mg/dl) in the mice (Fig. 13B). Further, diabetic state graphs illustrate the accelerated restoration to normoglycemia for mice with grafts containing the microporous scaffold with fibrin/PDGF (Fig. 13C).

To assess the *in vivo* efficacy of the scaffolds at restoring euglycemia in a chemically induced diabetic Lewis rat model, the rats were implanted with a scaffold loaded with 1500 IEQ in the omental pouch site (Fig. 14A) or 1500 IEQ free islets in the standard rodent kidney capsule transplant site. An intravenous glucose tolerance test was performed on functional graft recipients over 100 days post-transplant by measuring blood glucose at timepoints following the injection of a bolus of glucose. Islets loaded in a silicone scaffold and implanted in the omentum, free islets implanted in the omentum, and free islets implanted in the kidney capsule all illustrated similar glucose clearance profiles (Fig. 14C). After removal of the implanted cells at 168 days, hyperglycemia was restored, illustrating that the implants alone were responsible for the control of blood glucose levels (Fig. 14B). The scaffold was stained for islets (red) and nuclei (DAPI) after graft explanation, demonstrating that the islets were still viable and functional at the time of explanation (Fig. 14D).

The *in vivo* efficacy of the scaffolds in restoring euglycemia in an allogeneic streptozotocin (STZ)-diabetic mouse model was also assessed. Fibronectin-coated PDMS macroporous scaffolds with or without fibrin and PDGF, loaded with 3000 or 5000 IEQ allogeneic islets, were implanted into the omental pouch site of chemically induced diabetic Lewis rats. A control group received 3000 IEQ free islets into the standard kidney capsule transplant site. The scaffolds with fibrin and PDGF, seeded with allogeneic islets, were able to restore normoglycemia in the rats over a time course of at least 28 days (Fig. 14E).

To assess the *in vivo* efficacy of the scaffolds at restoring euglycemia in a diabetic baboon model, fasting blood glucose, post-prandial blood glucose and exogenous insulin levels were charted in a baboon (made diabetic by partial pancreatectomy and subsequent STZ administration) with 25,000 allogeneic IEQ/kg loaded within 6 PDMS macroporous scaffolds and implanted into the omental pouch. The baboon also received intravenous antirejection anti-CD154 monotherapy at 20 mg/kg on post-operative days 0, 1, 4, 10, 18, and 28 and every week thereafter as maintenance. At 364 days post-implant, the baboon continued to exhibit blood glucose control (Fig. 15).

Example 5: Release of Dexamethasone from macroporous silicone scaffolds or separate elements.

To test the release of agents from the scaffold, 10% or 20% w/v of dexamethasone was incorporated within two different sizes of PDMS macroporous scaffolds (8 mm and 10 mm diameter) by mixing the dexamethasone powder with the silicone polymer prior to loading into the molds. Elution of the dexamethasone was measured upon incubation of the scaffold in a buffer solution. The incorporated dexamethasone showed sustained release from the scaffold material over a time course of at least 12 days (Fig. 16).

Agents may also be incorporated into and released from elements separate from the macroporous scaffolds. Fig. 17 illustrates examples of potential drug-releasing geometries. Dexamethasone incorporated into PDMS disks at 0%, 5%, 10% or 20% w/v or a PDMS cage at 10% w/v, where the dexamethasone was mixed with the polymer prior to pouring into the respective molds, demonstrated sustained release properties over the course of the study (Fig. 18).

To examine the performance of agent-releasing elements *in vivo,* chemically induced diabetic mice were implanted subcutaneously with PDMS disks in which Dexamethasone was incorporated at 0%, 5%, 10% or 20% w/v. Dexamethasone in the urine of the mice was monitored over a time course and exhibited sustained release from the PDMS disks over the course of the study (Fig. 19A).

The effect of dexamethasone release on the performance of islets implanted in chemically induced diabetic mice was also examined. The mice were implanted with syngeneic islets within the macroporous scaffold at the epididymal fat pad site with zero, one or two 5% w/v dexamethasone releasing rods adjacent to the scaffold. No detrimental effect was observed in the time to reversal to normoglycemia in the presence of dexamethasone (Fig. 19B).

### Claims

1.  An implantable scaffold construct comprising:

    a) a macroporous scaffold fabricated from organosilicone and coated with fibronectin for providing structural support and spatial distribution to implanted cells;
    b) fibrin glue; and
    c) platelet-derived growth factor (PDGF) loaded into void spaces.

2.  The scaffold construct of claim 1, wherein said cells are insulin-producing cells.

3.  The scaffold construct of claim 1 or 2, wherein said scaffold has at least one property selected from:

    a) a porosity of 70-95%; and
    b) pore sizes of 75-400 $\mu$m.

4.  The scaffold construct of any one of claims 1-3, wherein the cells comprise mesenchymal stem cells.

5.  The scaffold construct of any one of claims 1-4 comprising implanted cells for use in the treatment of a disorder in a subject, wherein the implanted cells deliver a therapeutic benefit.

6.  The scaffold construct according to claim 5, wherein the disorder is diabetes.

### Patentansprüche

1.  Implantierbares Gerüstkonstrukt umfassend:

    a) ein makroporöses Gerüst, das aus Organosilikon hergestellt ist und mit Fibronectin beschichtet ist, zur Bereitstellung einer strukturellen Stütze und räumlicher Verteilung für implantierte Zellen;
    b) Fibrinkleber; und
    c) Blutplättchen-Wachstumsfaktor (platelet-derived growth factor; PDGF), der in leere Räume geladen wird.

2.  Gerüstkonstrukt nach Anspruch 1, wobei die Zellen Insulin-produzierende Zellen sind.

3.  Gerüstkonstrukt nach Anspruch 1 oder 2, wobei das Gerüst mindestens eine Eigenschaft hat, ausgewählt aus:

    a) einer Porosität von 70-95%; und
    b) Porengrößen von 75-400 $\mu$m.

4.  Gerüstkonstrukt nach einem der Ansprüche 1 bis 3, wobei die Zellen mesenchymale Stammzellen umfassen.

**5.** Gerüstkonstrukt nach einem der Ansprüche 1 bis 4, umfassend implantierte Zellen zur Verwendung bei der Behandlung einer Störung in einem Individuum, wobei die implantierten Zellen einen therapeutischen Nutzen bereitstellen.

**6.** Gerüstkonstrukt nach Anspruch 5, wobei die Störung Diabetes ist.

**Revendications**

**1.** Construction d'échafaudage implantable comprenant :

a) un échafaudage macroporeux fabriqué à partir d'organosilicium et revêtu de fibronectine pour fournir un support structurel et une distribution spatiale à des cellules implantées ;
b) de la colle de fibrine ; et
c) le facteur de croissance dérivé des plaquettes (PDGF) chargé dans les espaces vides.

**2.** Construction d'échafaudage selon la revendication 1, dans laquelle lesdites cellules sont des cellules productrices d'insuline.

**3.** Construction d'échafaudage selon la revendication 1 ou 2, dans laquelle ledit échafaudage possède au moins une propriété sélectionnée parmi :

a) une porosité de 70-95 % ; et
b) des tailles de pore de 75-400 $\mu$m.

**4.** Construction d'échafaudage selon l'une quelconque des revendications 1 à 3, dans laquelle les cellules comprennent des cellules souches mésenchymateuses.

**5.** Construction d'échafaudage selon l'une quelconque des revendications 1 à 4, comprenant des cellules implantées pour une utilisation dans le traitement d'un trouble chez un sujet, où les cellules implantées offrent un bénéfice thérapeutique.

**6.** Construction d'échafaudage selon la revendication 5, où le trouble est le diabète.

**Figure 1**

**Figure 2**

Figure 3

**Figure 4**

A

B

Figure 5

Figure 6

**A**

Day 0

**B**

Day 0

# Figure 7

A

B

C

Figure 8

Figure 9

Figure 10

A

| Islet Diameter (μm): | 50-100 | 100-150 | 150-200 | 200-250 | 250-300 | 300-350 |
|---|---|---|---|---|---|---|
| Average % Distriubtion of Islets by Size: | 74% | 21% | 4% | 1% | 0% | 0% |

B

| Islet Diameter (μm): | 50-100 | 100-150 | 150-200 | 200-250 | 250-300 | 300-350 | 360-400 |
|---|---|---|---|---|---|---|---|
| Average % Distriubtion of Islets by Size: | 28% | 40% | 20% | 7% | 2% | 1% | 2% |

# Figure 11

Figure 12

Figure 13

C

Figure 13 (cont.)

A

B

# Figure 14

C

D

Figure 14 (cont.)

E

Figure 14 (cont.)

Figure 15

Figure 16

A

B

Figure 17

Figure 18

Figure 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MINQUEZ-CASTELLANOS et al.** *J Neurol Neurosurg Psychiatry,* 2007
- **CHANG et al.** *Trends Biotechnol,* 1999, vol. 17, 78-83
- **RINSCH et al.** *Kidney Intern,* 2002, vol. 62, 1395-1401
- **ZHENG et al.** *immunity,* 2003, vol. 19 (4), 503-514
- **MALCOLM, K. et al.** *J. Contr. Rel.,* 2003, vol. 90, 217
- **BECK et al.** *Tissue Eng,* 2007, vol. 13 (3), 1-11